# EUROPEAN PATENT APPLICATION

(11) **EP 1 582 162 A1**
(43) Date of publication of application: **05.10.2005**
(21) Application number: 04007788.5
(22) Date of filing: 31.03.2004
(51) Int. Cl.: A61B 17/72

(54) **Intramedullary nail provided with expansion fixing means comprising at least one element of shape-retention material**

(71) Applicant: ORTHOFIX INTERNATIONAL B.V., 1071 Amsterdam (NL)
(72) Inventor: Coati, Michele, 37029 San Pietro in Cariano (Verona) (IT); Marazzi, Giancarlo, 21047 Saronno (Varese) (IT); Marini, Graziano, 37060 Castel D'Azzano (Verona) (IT); Rossi, Graziano, 37139 Verona (IT); Rossi, Luigi, 37019 Peschiera de Garda (Verona) (IT); Venturini, Daniele, 37064 Povegliano Veronese (Verona) (IT)
(74) Representative: Ferreccio, Rinaldo

(57) **Abstract**

An intramedullary nail (10, 110, 210, 310, 410, 510, 610, 710) suitable for insertion in a fractured elongate bone (12) and for an unusually simple fixation therein comprises a substantially straight stem (14) extending between a proximal end (16) and a distal end (18), said stem (14) comprising at least a substantially longitudinal distribution (20) of expansion means for the nail fixation to the bone, said expansion means comprising at least one element (22) realised with a shape-memory material, a free end (24) of said at least one element (22) positioning outside the stem (14) to perform said fixation.

## Description

### Field of application

The present invention relates in its more general aspect to an intramedullary nail suitable for insertion in a fractured elongate bone and an application method of said nail in said bone.

In particular, the invention relates to an intramedullary nail suitable for insertion in a fractured elongate bone, such as a femur or a tibia, comprising a substantially straight stem, having a predetermined axis, extending between a proximal end and a distal end.

### Prior art

Intramedullary nails are known, which, during a surgical operation, are inserted in a fractured elongate bone and fixed therein, in order to reconstruct the original bone configuration and meanwhile recover the bone solidity, so that callus regeneration mechanisms can correctly occur.

The stems of these intramedullary nails are generally cylinder-shaped and they can be both solid and hollow.

In order to fix the intramedullary nail to the bone portions to be reconstructed, two offset holes are usually provided on the nail, having axes lying on parallel planes and extending diametrically across the stem, in correspondence with the nail distal end, and two offset holes having the same size, having axes not necessarily lying on parallel planes, in correspondence with the nail proximal end. Said holes are suitable for housing bone screws, which are inserted, after a convenient bone drilling, in the bone, with the subsequent fixation of the intramedullary nail to the bone portions.

Although advantageous under different points of view, intramedullary nails being structured as above schematically described have known drawbacks mainly underlined when bone drillings are to be performed for bone screw insertion. This step is particularly critical since it is known that a good nail fastening essentially depends on the correct realisation of these bone drillings, obviously made in correspondence with the holes of the inserted intramedullary nail.

However the precise location of the intramedullary nail holes is made difficult by the fact that the holes are no more visible, being the nail inserted in the bone. It is then worth underlining a further location problem, i.e. the fact that the intramedullary nail can be, when being inserted, slightly bent, so that the holes at the nail distal end are no more, with respect to the proximal end, in the same position as before installing the nail.

The traditional technique for locating the holes of an inserted intramedullary nail provides the use of X rays, involving however the well known risks of cumulative exposure of the operating staff, besides being quite awkward during the surgical operation.

Specific mechanical devices have thus been studied and realised, such as for example the one described in the European patent no. EP 772 420 in the name of the same Applicant.

These devices do not require the use of X rays, but they have the drawback of requiring several operational steps, all to be performed quite carefully and precisely.

### Summary of the invention

The problem underlying the present invention is to provide an intramedullary nail suitable for insertion in a fractured elongate bone, capable to meet the above-mentioned requirement, meanwhile overcoming, in a simple and effective way, all the drawbacks mentioned with reference to the prior art.

This problem is solved, according to the present invention, by an intramedullary nail suitable for insertion in a fractured elongate bone, as above described and characterised in that said stem comprises at least a substantially longitudinal distribution of expansion means for the nail fixation to the bone, said expansion means comprising al least one element realised with a shape-memory material, a free end of said at least one element positioning outside the stem to perform said fixation.

Further features and the advantages of the intramedullary nail suitable for insertion in a fractured elongate bone, as well as of the application method of said nail in said fractured bone, according to the present invention, will be apparent from the following description of an embodiment thereof made with reference to the attached drawings, given by way of non-limiting example.

### Brief description of the drawings

Figure 1 is a schematic perspective view of an intramedullary nail suitable for insertion in a fractured elongate bone, according to the present invention.
Figure 2 is a schematic front-elevation view of the nail of figure 1.
Figure 3 is a schematic front-elevation view of a first alternative embodiment of the nail of figure 2.
Figure 4 is a schematic perspective view of a second alternative embodiment of an intramedullary nail according to the present invention.
Figure 5 is a schematic front-elevation view of the nail of figure 4.
Figure 6 is a schematic front-elevation view of a third alternative embodiment of an intramedullary nail according to the present invention.
Figure 7 is a schematic perspective view of a forth alternative embodiment of an intramedullary nail according to the present invention.
Figure 8 is a schematic perspective view of a fifth alternative embodiment of an intramedullary nail according to the present invention.
Figure 9 is a schematic perspective view of a sixth alternative embodiment of an intramedullary nail according to the present invention.
Figure 10 is a schematic perspective view of the intramedullary nail of figure 9, in a different configuration.
Figure 11 is a schematic perspective view of a seventh alternative embodiment of an intramedullary nail according to the present invention.

### Detailed description

With reference to the drawings, an intramedullary nail according to the present invention is shown in a first embodiment and in seven further alternative embodiments.

The nail, in the embodiment described at first, is globally indicated with 10, while in the alternative embodiments the nail of the invention is indicated with 110, 210, 310, 410, 510, 610 and 710 respectively. It is specified that, in these alternative embodiments, the elements being structurally or functionally similar to the elements of the nail 10 are indicated with the same reference number and the detailed description thereof is not repeated for short.

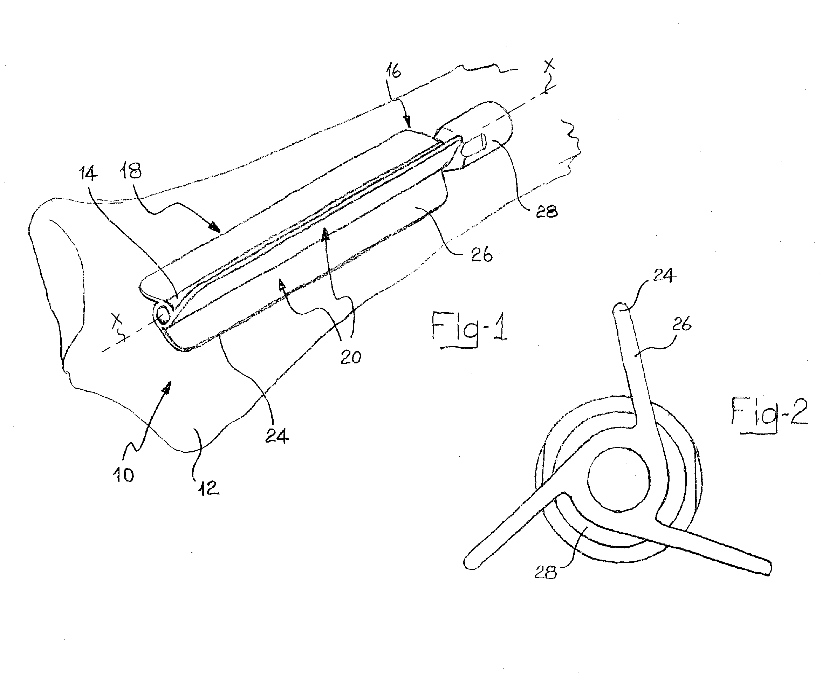

The nail 10 shown in figures 1 and 2, which is suitable for insertion in a fractured elongate bone 12, such as for example a femur or a tibia, comprises a substantially straight stem 14 extending between a proximal end 16 and a distal end 18. The stem is preferably composed of a cylindrical tubular body.

According to an aspect of the present invention, the stem 14 comprises at least a substantially longitudinal distribution 20 of expansion means for the nail fixation to the bone. The substantially longitudinal distribution 20 comprises at least an element 22 realised with a shape-memory material, a free end 24 of said at least one element 22 positioning outside the stem 14 to perform said fixation.

Shape memory material means a material having a given initial starting shape and taking, under predetermined external conditions (for example a temperature rise and/or drop) or undergoing a predetermined activation condition, i.e. after a so-called "material instruction" step, a given new shape.

Known shape-memory materials, which are suitable for use in the present invention, are, for instance, certain nickel-titanium alloys.

The invention is based on the following principle: subjecting an element realised in a shape memory material, having a predetermined initial shape at rest, to said so-called "material instruction" step (for example to a predetermined temperature variation), said element takes a shape being maintained as long as the "instruction" step effect persists; said shape, being different from the initial shape, can be called transient shape and it is temporary or unstable. When the "instruction" step effect stops, the element leaves said transient shape and, coming back towards the initial shape, takes a working shape. Depending on the type of material and on the working temperature, in the working shape, said material can arrive at the initial shape or it can go further on the initial shape, arriving at a final shape. It is worth pointing out that the more this final shape is far from the initial shape, the more the available shape memory energy of the material is high.

The invention has been reached as result of the intuition that an element realised in a shape memory material, in the passage from the transient shape towards the working shape, can create the fixing of the intramedullary nail in the bone.

In the example of figures 1 and 2, the substantially longitudinal distributions 20 are three and they are angularly equally spaced around the side surface of the cylindrical tubular body of the stem 14

The element 22 made of a shape-memory material of each distribution 20 shapes in a substantially rectangular tongue 26, with the longest side being longitudinally developed with respect to the stem 14 and about as long as the stem 14. The tongue 26 is connected outside the stem 14 by a longest side thereof, while the opposite longest side is the free end 24 of said element 22.

In the final shape, i.e. when the shape-memory material is activated, the tongues 26 are positioned in a substantially tangential way with respect to the cylindrical tubular body of the stem 14, the tongues 26 being all positioned with a same rotation direction, i.e. the free ends 24 of the tongues 26 being all for example arranged in an angular position preceding (once the clockwise direction is set) the angular position of the longest side connected to the stem 14.

In the initial shape the tongue 26 is bent towards the side surface of the stem 14, it abuts for example on the side surface of the stem 14, and according to the width thereof, it can partially abuts on the adjacent tongue 26 (i.e. the tongue being arranged in an angular position preceding the angular position of the tongue 26 being concerned, the clockwise direction being always set).

The tongues 26 have connection arcs with the stem 14 being such as to reduce the stress concentration; meanwhile, these arcs have a limited extension, since it is worth allowing the deformation of most of each tongue 26.

The stem 14, at one end, has a head 28 having a substantially cylindrical shape and an axis substantially coinciding with the stem 14 axis: said head 28 has traditional means for hooking the nail 10, during the insertion thereof in the bone 12, with a traditional external guide device.

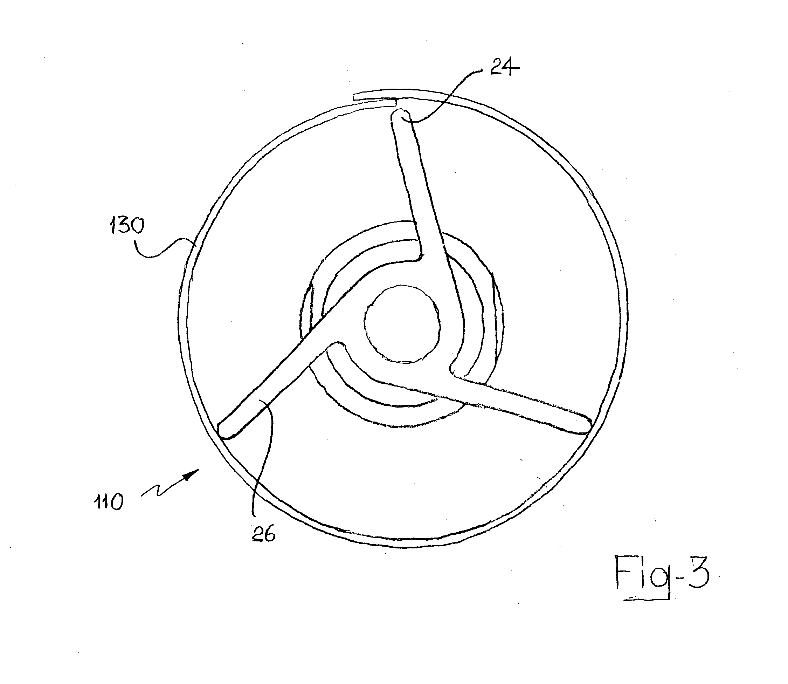

In the alternative embodiment of figure 3, the nail 110 according to the invention is shown, having a similar structure to the structure of the nail 10 of figure 1, with an additional covering element 130 enveloping the tongues 26 of the stem 14.

The covering element 130 shapes in a flat spiral spring which is preferably constrained to the free end 24 of a tongue 26.

The flat spiral spring, being longitudinally developed with respect to the stem 14, has a rest inner diameter being substantially equal to the cylindrical dimension of the tongues 26 in the initial position thereof. When the tongues 26 are operated, the inner diameter of the spiral spring is expanded according to the position taken by the free ends 24 of the three tongues 26.

Preferably, the spiral spring has outwardly a considerably rough surface.

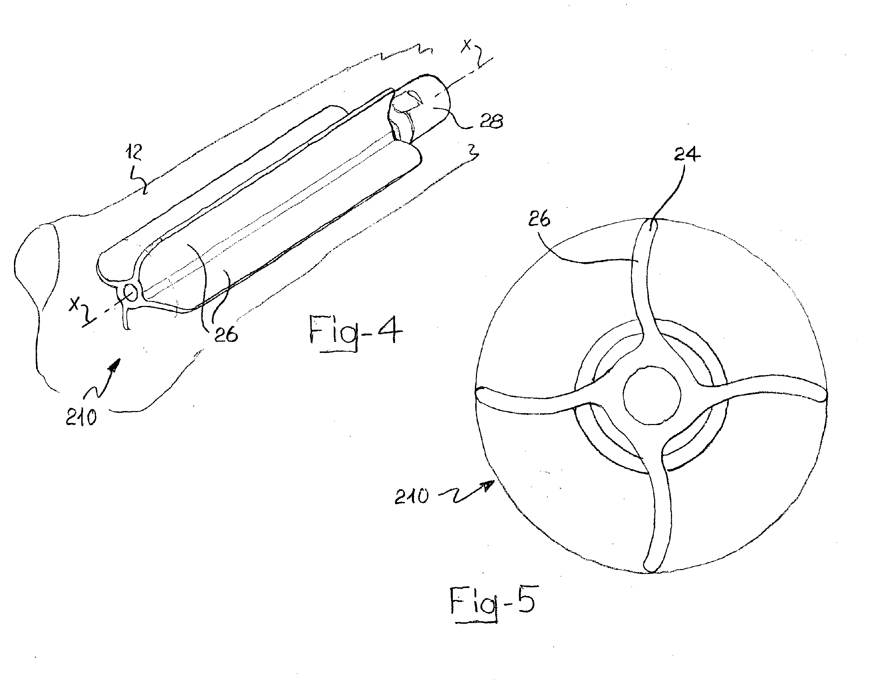

In the alternative embodiment of figures 4 and 5 the nail 210 according to the invention is shown, wherein the substantially longitudinal distributions 20 are four and they are angularly equally spaced around the side surface of the cylindrical tubular body of the stem 14

Similarly to the nail 10, the element 22 made of a shape-memory material of each distribution 20 shapes in a substantially rectangular tongue 26, with the longest side being longitudinally developed with respect to the stem 14 and about as long as the stem 14. The tongue 26 is connected outside the stem 14 by a longest side thereof, while the opposite longest side is the free end 24 of said element 22.

In the final shape, i.e. when the shape-memory material is activated, the tongues 26 are positioned in a substantially tangential way with respect to the cylindrical tubular body of the stem 14, the tongues 26 being all positioned with a same rotation direction, i.e. the free ends 24 of the tongues 26 being all for example arranged in an angular position preceding (once the clockwise direction is set) the angular position of the longest side connected to the stem 14.

In the final shape shown in figures 4 and 5, the tongues 26 are bent outside the stem 14: more precisely, the tongue concavity is the one deriving from the fact that the free end 24 of the tongue 26 is arranged in an angular position following (once the clockwise direction is set) the angular position of the respective tangent to the cylindrical tubular body of the stem 14.

In the initial shape the tongue 26 is bent towards the side surface of the stem 14, it abuts for example on the side surface of the stem 14, and according to the width thereof, it can partially abuts on the adjacent tongue 26 (i.e. the tongue being arranged in an angular position preceding the angular position of the tongue 26 being concerned, the clockwise direction being always set).

The tongues 26 have connection arcs with the stem 14 being such as to reduce the stress concentration; meanwhile, these arcs have a limited extension, since it is worth allowing the deformation of most of each tongue 26.

The stem 14, at one end, has a head 28 having a substantially cylindrical shape and an axis substantially coinciding with the stem 14 axis: said head 28 has traditional means for hooking the nail 10, during the insertion thereof in the bone 12, with a traditional external guide device.

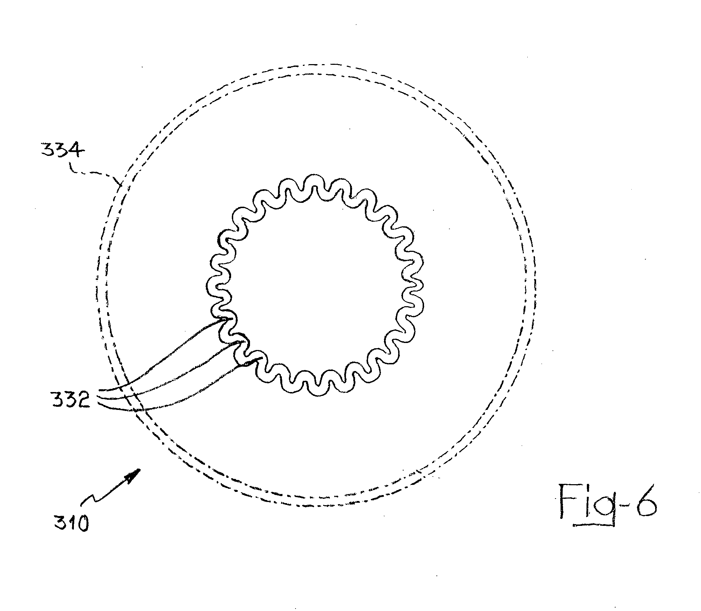

In the alternative embodiment of figure 6 the nail 310 according to the invention is shown, wherein the elements 22 made of a shape-memory material of the substantially longitudinal distributions 20 shape in a plurality of corrugations 332 of a tubular structure 334 realised with a shape-memory material. The corrugations 332 are continuously distributed along the side surface of the tubular structure 334, which is internally constrained to the cylindrical tubular body of the stem 14. Alternatively, the tubular structure 334 is self load-bearing.

More precisely, the tubular structure 334 undergoes an instruction step of the shape-memory material wherein, starting from a tubular structure with a circular outer profile (final shape of the nail 310), a tubular structure with a more reduced outer dimension is obtained (initial shape of the nail 310), by forming successive corrugations 332.

It is evident that in this alternative embodiment the free ends 24 of the corrugations 332 are practically the grooves of the corrugations 332, which, in the final shape of the nail 310, are positioned outside the initial dimension of the tubular structure 334, to perform the nail fixation to the bone.

By way of example, the outer diameter of the tubular structure 334 in the final shape (shown with dotted lines in the figure) can be of 20 mm, while the thickness of the tubular structure 334 can be of 0,4 mm.

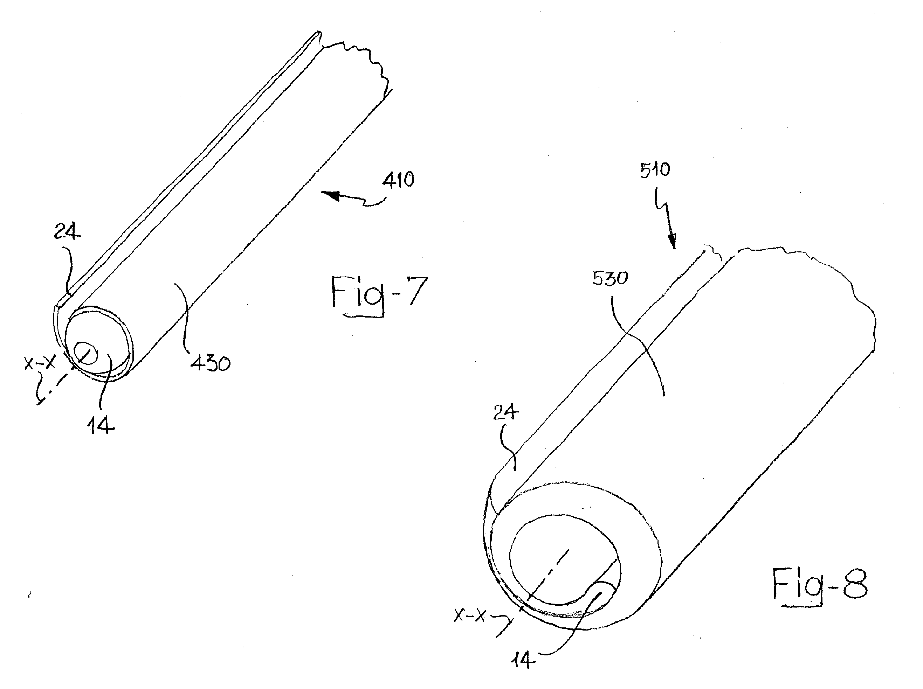

In the alternative embodiment of figure 7 the nail 410 according to the invention is shown, wherein the element 22 made of a shape-memory material of the substantially longitudinal distribution 20 shapes in a covering element 430 enveloping the stem 14.

The covering element 430 shapes in a flat-spiral-shaped bent plate.

An end of said plate is connected to the stem 14 through traditional connection means, such as welding or mechanical joint, so to be substantially tangent to the cylindrical tubular body of the stem 14.

An opposite end of the bent plate is the free end 24 of the shape-memory element 22. In the final shape, this free end 24 is positioned, with respect to the stem 14, far outside than in the initial shape of the nail 410. In other words, the plate flat spiral enlarges, i.e. it radially expands when passing from the initial shape to the final shape.

The bent plate can also be split into pieces, to limit the risk of rotation of the nail 410 in the bone in the plate expansion step.

In the alternative embodiment of figure 8 the nail 510 according to the invention is shown, wherein the element 22 made of a shape-memory material of the substantially longitudinal distribution 20 shapes in a covering element 530 enveloping the stem 14.

The covering element 530 shapes in a flat-spiral-shaped bent plate.

An end of said plate is connected to the stem 14 through traditional connection means, such as welding or mechanical joint, so that the flat spiral strays in a substantially radial direction with respect to the stem 14 and it is preferably developed for about a turn and a half.

An opposite end of the bent plate is the free end 24 of the shape-memory element 22. In the final shape, this free end 24 is positioned, with respect to the stem 14, far outside than in the initial shape of the nail 510. In other words, the plate flat spiral enlarges, i.e. it radially expands when passing from the initial shape to the final shape.

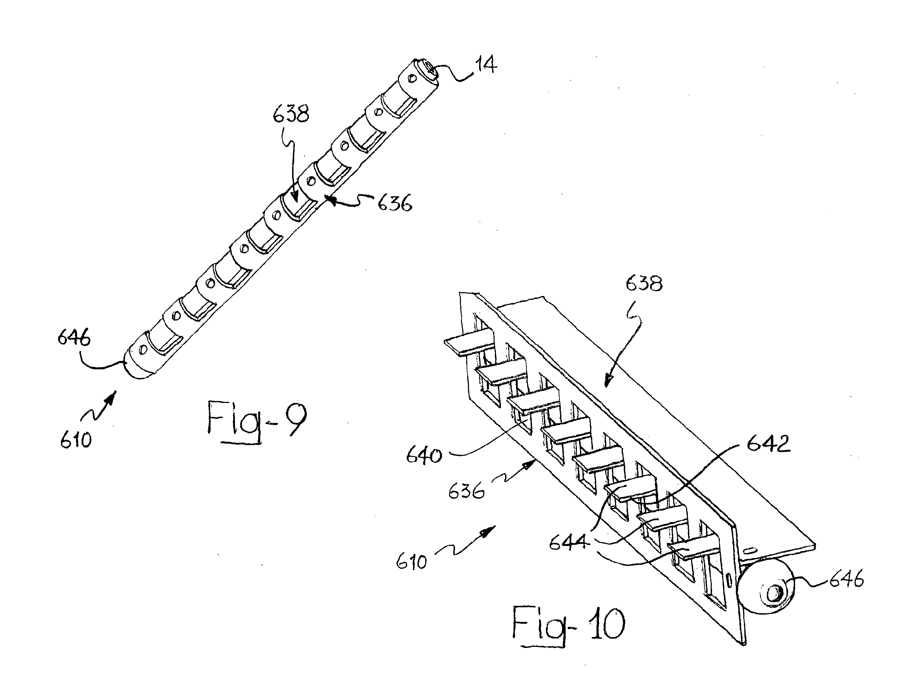

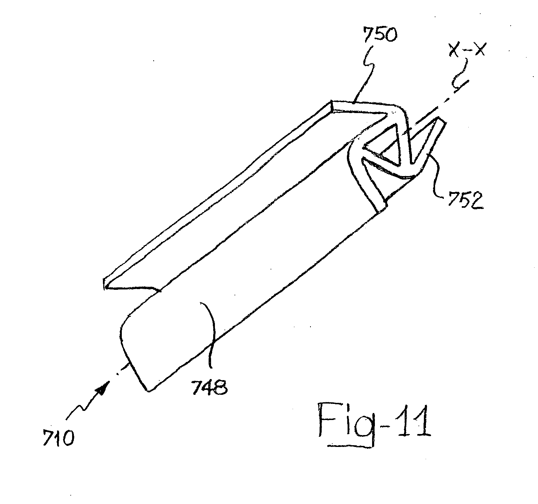

In the alternative embodiment of figures 9 and 10 the nail 610 according to the invention is shown, wherein the substantially longitudinal distributions 20 shape in two shaped plates 636 and 638, realised with a shape-memory material.

The shaped plates 636 and 638, in the final shape, are substantially flat and they are substantially rectangle-shaped, with the longest side being developed with the axis of the stem 14 and about as long as the stem 14.

More particularly, each of said shaped plates 636 and 638 is connected to the stem 14, along an intermediate line between the rectangle longest sides of the respective plates 636 and 638, in a substantially tangential way to the stem 14, for example by welding.

The intermediate connection lines are positioned substantially parallel to the axis of the stem 14, and they are for example offset on the outer surface of the stem 14 by about a right angle.

The planes of the two so-arranged plates 6363 and 638 intersect: respective material lacks 640 and 642 allowing such an arrangement are thus provided.

More precisely, in the first shaped plate 636, these lacks 640 shape in rectangular slits, positioned in series in the direction of the longest side of the rectangle of the plate 636. In the second shaped pate 638, these lacks 642 shape in rectangular slots, positioned in series in the direction of the longest side of the rectangle of the plate 638. The slots are realised starting from a longest side of the rectangle of the plate 638 and they thus define a series of teeth 644, being positioned in correspondence with the rectangular slits and having suitable dimensions for passing through these slits.

The above description corresponds to the final shape of the two shaped plates 636 and 638 having thus globally four series of free ends 24 for the fixation and i.e. the two longest sides of the rectangle of the plate 636, the teeth 644 and the longest side of the rectangle of the plate 638 being opposite to the teeth 644.

In the initial shape, the two plates 636 and 638 are bent with the concavity towards the stem 14; in other words, they lie enveloped on the outer surface of the stem 14.

By way of example, in the final shape, the plates 636 and 638, intersecting with each other as above described, have an outer dimension equal to a cylinder with a diameter of 19 mm.

It must be noticed that it is possible to have two plates 636 and 638 with free ends 24 distributed only in some areas (for example close to the ends of the stem 14), conveniently selecting for example the position of the teeth 644.

A ferrule 646 is preferably positioned at one end of the stem 14 to improve the insertion of the nail 610. This ferrule shapes in an ellipsoid portion, centrally drilled in correspondence with the axis of the stem 14.

In the alternative embodiment of figure 11 the nail 710 according to the invention is shown, wherein the substantially longitudinal distributions 20 shape in three plates 748, 750 and 752, realised with a shape-memory material.

In the final shape, the three plates 748, 750 and 752 are substantially flat and rectangular, with the longest side along the axis of the nail 710.

They are connected to each other, by means of welds and/or joints. The connection occurs by joining a longest side of a plate to an intermediate and parallel line to the longest sides of the rectangle of the adjacent plate. In other words, an equilateral-triangle-shaped section exists, with each of the three sides extending on a same side with respect to the single side being considered.

In the initial shape, the plates 748, 750 and 752 are bent in correspondence with the respective intermediate lines of the mutual connection, so that, reasoning in the section terms, the extension of each side of the equilateral triangle is bent towards the side connected to the side being considered.

The stem 14 is inserted in the three so-connected plates 748, 750 and 752 and it is constrained thereto.

An application method of an intramedullary nail according to the present invention in said fractured elongate bone 12 comprises:
a positioning step of said nail in said elongate bone 12, to mend the fracture;
an operation step of said at least one substantially longitudinal distribution of expansion means for the nail fixation to the bone, said at least one substantially longitudinal distribution of expansion means being operated by said at least one element realised with a shape-memory material.

The operation of the intramedullary nail, suitable for insertion in a fractured elongate bone 12, according to the present invention, is already partially evident from the description of the above application method and it is specified hereafter.

The nail according to the invention is positioned in said elongate bone 12, in the initial shape thereof, the stem 14 playing a structural role.

Afterwards, in order to fix the nail to the bone 12, said at least one element 22 realised with a shape-memory material is operated: the free ends 24 of said at least one substantially longitudinal distribution 20 of expansion means tend to be positioned outside the stem 14 to realise an interference between said free ends 24 and the bone portion surrounding them. This interference practically causes a grip, solidly fixing the nail in the bone 12.

The main advantage achieved by the intramedullary nail suitable for insertion in a fractured elongate bone, as well as by the application method of said nail in said fractured bone, according to the present invention, is the fact of unusually simplifying the fixation step of the intramedullary nail inserted in the fractured bone. In practise, the nail fixation is obtained simultaneously with the insertion thereof in the bone, since the shape-memory elements, cooled at first to take the initial shape, take in the bone their final shape, due to the heat released by the patient's body.

Another advantage of the intramedullary nail according to the present invention, is to prevent undesired nail torsions or rotations in the bone during the application step, due to the structural continuity of the described embodiments.

A further advantage of the intramedullary nail according to the present invention is that the stem in the shape of a cylindrical tubular body allows an easy insertion in the bone by using a guide wire.

In the case of the nail 10, it must be noticed that the load applied on the medullary canal of the bone 12 by the three tongues 26 is advantageously distributed along the circumference of the stem 14.

The shortest side of the rectangle of the tongues 26 can be very long, exploiting all the space of the stem 14 lying between one tongue and the other.

The head 28 allows the shape-memory tongues 26 to be slightly below the insertion point of the nail 10.

In the case of the nail 110, it must be noticed that the spiral spring has outwardly a considerably rough surface to ensure a high friction between the spring and the bone 12 when in the fixation conditions of the nail 110.

In the case of the nail 210, it must be noticed that the tongues 24 are bent to better exploit the shape-memory material deformation.

The heat 28 allows the shape-memory tongues 26 to be slightly below the insertion point of the nail 210.

In the case of the nail 310, it must be noticed that, advantageously, the radial thrust of the tubular structure 334 is almost uniform along the length of the nail 310.

In the case of the nail 710, it must be noticed how advantageously the nail is structurally particularly simple.

Obviously, in order to meet specific and contingent requirements, a skilled in the art could bring several changes to the above-described intramedullary nail suitable for insertion in a fractured elongate bone and application method of said nail in said bone, all however comprised in the scope of protection of the present invention, as defined in the following claims.

## Claims

1. Intramedullary nail (10, 110, 210, 310, 410, 510, 610, 710) suitable for insertion in a fractured elongate bone (12), comprising a substantially straight stem (14) extending between a proximal end (16) and a distal end (18), **characterised in that** said stem (14) comprises at least a substantially longitudinal distribution (20) of expansion means for the nail fixation to the bone, said expansion means comprising at least one element (22) realised with a shape-memory material, a free end (24) of said at least one element (22) positioning outside the stem (14) to perform said fixation.

2. Intramedullary nail (10, 110, 210, 310, 410, 510, 610, 710) according to claim 1, **characterised in that** the stem (14) is composed of a cylindrical tubular body.

3. Intramedullary nail (10) according to claim 2, **characterised in that** the substantially longitudinal distributions (20) are three and they are angularly equally spaced around the side surface of the cylindrical tubular body of the stem (14).

4. Intramedullary nail (10) according to claim 3, **characterised in that** the element (22) made of a shape-memory material of each distribution (20) shapes in a substantially rectangular tongue (26), with the longest side being longitudinally developed with respect to the stem (14) and about as long as the stem (14).

5. Intramedullary nail (10) according to claim 4, **characterised in that** the tongue (26) is connected outside the stem (14) by a longest side thereof, the opposite longest side being the free end (24) of said element (22).

6. Intramedullary nail (10) according to claim 5, **characterised in that**, in the shape corresponding to the fixation of the nail (10), the tongues (26) are positioned in a substantially tangential way with respect to the cylindrical tubular body of the stem (14), the tongues (26) being all positioned with a same rotation direction.

7. Intramedullary nail (10) according to claim 5, **characterised in that**, in the shape being suitable for the insertion of the nail (10) in the bone, the tongue (26) is bent towards the side surface of the stem (14).

8. Intramedullary nail (10) according to claim 5, **characterised in that**, in the shape being suitable for the insertion of the nail (10) in the bone, the tongue (26) is bent towards the side surface of the stem (14) and it partially abuts on the adjacent tongue (26).

9. Intramedullary nail (10) according to claim 5, **characterised in that** the tongues (26) have connection arcs with the stem (14) being such as to reduce the stress concentration.

10. Intramedullary nail (10) according to claim 5, **characterised in that** the tongues (26) have connection arcs having a limited extension to allow the deformation of most of each tongue (26).

11. Intramedullary nail (10, 210) according to claim 1, **characterised in that** the stem (14), at one end, has a head (28) having a substantially cylindrical shape and an axis substantially coinciding with the axis of the stem (14).

12. Intramedullary nail (10, 210) according to claim 1, **characterised in that** said head (28) has traditional means for hooking the nail (10), during the insertion thereof in the bone (12), with an external guide device.

13. Intramedullary nail (110) according to claim 5, **characterised in that** it provides a covering element (130) enveloping the tongues (26) of the stem (14).

14. Intramedullary nail (110) according to claim 13, **characterised in that** the covering element (130) shapes in a flat spiral spring, longitudinally developed with respect to the stem (14).

15. Intramedullary nail (110) according to claim 14, **characterised in that** said flat spiral spring is constrained to the free end (24) of a tongue (26).

16. Intramedullary nail (110) according to claim 14, **characterised in that** said flat spiral spring has a rest inner diameter being substantially equal to the cylindrical dimension of the tongues (26) in the position thereof preceding the nail insertion in the bone.

17. Intramedullary nail (110) according to claim 14, **characterised in that** said flat spiral spring has outwardly a considerably rough surface.

18. Intramedullary nail (210) according to claim 2, **characterised in that** the substantially longitudinal distributions (20) are four and they are angularly equally spaced around the side surface of the cylindrical tubular body of the stem (14).

19. Intramedullary nail (210) according to claim 18, **characterised in that** the element (22) made of shape-memory material of each distribution (20) shapes in a substantially rectangular tongue (26), with the longest side being longitudinally developed with respect to the stem (14) and about as long as the stem (14).

20. Intramedullary nail (210) according to claim 19, **characterised in that** the wing (26) is connected outside the stem (14) by a longest side thereof, the opposite longest side being the free end (24) of said element (22).

21. Intramedullary nail (210) according to claim 20, **characterised in that**, in the shape corresponding to the fixation of the nail (10), the tongues (26) are positioned in a substantially tangential way with respect to the cylindrical tubular body of the stem (14), the tongues (26) being all positioned with a same rotation direction.

22. Intramedullary nail (210) according to claim 21, **characterised in that**, in the shape corresponding to the fixation of the nail (10), the tongues (26) are bent outside the stem (14).

23. Intramedullary nail (210) according to claim 22, **characterised in that** the tongue concavity is the one deriving from the fact that the free end (24) of the tongue (26) is arranged in an angular position following, once the clockwise direction is set, the angular position of the respective tangent to the cylindrical tubular body of the stem (14).

24. Intramedullary nail (210) according to claim 20, **characterised in that**, in the shape preceding the nail insertion in the bone, the tongue (26) is bent towards the side surface of the stem (14).

25. Intramedullary nail (210) according to claim 20, **characterised in that**, in the shape preceding the nail insertion in the bone, the tongue (26) is bent towards the side surface of the stem (14) and it partially abuts on the adjacent tongue (26).

26. Intramedullary nail (210) according to claim 20, **characterised in that** the tongues (26) have connection arcs with the stem (14) being such as to reduce the stress concentration.

27. Intramedullary nail (210) according to claim 20, **characterised in that** the tongues (26) have connection arcs having a limited extension to allow the deformation of most of each tongue (26).

28. Intramedullary nail (310) according to claim 1, **characterised in that** the elements (22) made of a shape-memory material of the substantially longitudinal distributions (20) shape in a plurality of corrugations (332) of a tubular structure (334) realised with a shape-memory material.

29. Intramedullary nail (310) according to claims 2 and 28, **characterised in that** the corrugations (332) are discontinuously distributed along the side surface of the tubular structure (334), which is internally constrained to the cylindrical tubular body of the stem (14), the free ends (24) of the corrugations (332) being the grooves of the corrugations (332).

30. Intramedullary nail (310) according to claim 29, **characterised in that** the outer diameter of the tubular structure (334) in the shape corresponding to the nail fixation is about 20 mm, the thickness of the tubular structure (334) measuring about 0,4 mm.

31. Intramedullary nail (410, 510) according to claim 1, **characterised in that** the element (22) made of shape-memory material of the substantially longitudinal distribution (20) shapes in a covering element (430, 530) enveloping the stem (14).

32. Intramedullary nail (410, 510) according to claim 31, **characterised in that** the covering element (430, 530) shapes in a flat-spiral-shaped bent plate.

33. Intramedullary nail (410) according to claims 2 and 32, **characterised in that** an end of said plate is connected to the stem (14) through connection means so to be substantially tangent to the cylindrical tubular body of the stem (14).

34. Intramedullary nail (410) according to claim 33, **characterised in that** an opposite end of the bent plate is the free end (24) of the shape-memory element (22), in the shape corresponding to the hail fixation in the bone, said free end (24) being positioned, with respect to the stem (14), far outside than in the shape preceding the nail insertion.

35. Intramedullary nail (410) according to claim 32, **characterised in that** the bent plate is split into pieces.

36. Intramedullary nail (510) according to claim 32, **characterised in that** an end of said plate is connected to the stem (14) through connection means so that the flat spiral strays in a substantially radial direction with respect to the stem (14).

37. Intramedullary nail (510) according to claim 36, **characterised in that** the flat spiral is developed for about a turn and a half.

38. Intramedullary nail (510) according to claim 37, **characterised in that** an opposite end of the bent plate is the free end (24) of the shape-memory element (22), in the shape corresponding to the nail fixation in the bone, said free end (24) being positioned, with respect to the stem (14), far outside than in the shape preceding the nail insertion.

39. Intramedullary nail (610) according to claim 1, **characterised in that** the substantially longitudinal distributions (20) shape in two shaped plates (636, 638), realised with a shape-memory material.

40. Intramedullary nail (610) according to claim 39, **characterised in that** the shaped plates (636, 638), in the shape corresponding to the nail fixation, are substantially flat and substantially rectangle-shaped, with the longest side being developed with the axis of the stem (14) and about as long as the stem (14).

41. Intramedullary nail (610) according to claim 40, **characterised in that** each of said shaped plates (636, 638) is connected to the stem (14), along an intermediate line between the longest sides of the rectangle of the respective plates (636, 638), in a substantially tangential way to the stem (14).

42. Intramedullary nail (610) according to claim 41, **characterised in that** the intermediate connection lines are positioned substantially parallel to the axis of the stem (14).

43. Intramedullary nail (610) according to claim 41, **characterised in that** the intermediate connection lines are offset on the outer surface of the stem (14) by about a right angle.

44. Intramedullary nail (610) according to claim 41, **characterised in that** the planes of the two plates (636, 638) intersect, respective material lacks (640, 642) allowing such an arrangement being provided.

45. Intramedullary nail (610) according to claim 44, **characterised in that**, in the first shaped plate (636), said lacks (640) shape in rectangular slits, positioned in series in the direction of the longest side of the rectangle of the plate (636).

46. Intramedullary nail (610) according to claim 45, **characterised in that**, in the second shaped plate (638), said lacks (642) shape in rectangular slots, positioned in series in the direction of the longest side of the rectangle of the plate (638), the slots being realised starting from a longest side of the rectangle of the plate (638) and defining a series of teeth (644) being positioned in correspondence with the rectangular slits and having suitable dimensions for passing through said slits.

47. Intramedullary nail (610) according to claim 46, **characterised in that** the two shaped plates (636, 638) have globally four series of free ends (24) for the fixation and i.e. the two longest sides of the rectangle of the first plate (636), the teeth (644) and the longest side of the rectangle of the second plate (638) being opposite to the teeth (644).

48. Intramedullary nail (610) according to claim 46, **characterised in that**, in the shape preceding the nail insertion, the two plates (636, 638) are bent with the concavity towards the stem (14).

49. Intramedullary nail (610) according to claim 47, **characterised in that**, in the shape corresponding to the nail fixation, the plates (636, 638) have an outer dimension being equal to a cylinder with a diameter of about 19 mm.

50. Intramedullary nail (610) according to claim ,39, **characterised in that** the two plates (636, 638) have free ends (24) distributed only in some areas of the stem (14).

51. Intramedullary nail (610) according to claim 39, **characterised in that** a ferrule (646) is preferably positioned at one end of the stem (14) to improve the insertion of the nail (610).

52. Intramedullary nail (610) according to claim 51, **characterised in that** said ferrule shapes in an ellipsoid portion, centrally drilled in correspondence with the axis of the stem (14).

53. Intramedullary nail (710) according to claim 1, **characterised in that** the substantially longitudinal distributions (20) shape in three plates (748, 750, 752), realised with a shape-memory material.

54. Intramedullary nail (710) according to claim 53, **characterised in that**, in the shape corresponding to the nail fixation, the three plates (748, 750, 752) are substantially flat and rectangular, with the longest side along the axis of the nail (710).

55. Intramedullary nail (710) according to claim 54, **characterised in that** the three plates (748, 750, 752) are connected to each other, the connection occurring by joining a longest side of a plate to an intermediate and parallel line to the longest sides of the rectangle of the adjacent plate.

56. Intramedullary nail (710) according to claim 55, **characterised in that**, in the shape preceding the nail insertion, the plates (748, 750, 752) are bent in correspondence with the respective intermediate lines of the mutual connection.

57. Intramedullary nail (710) according to claim 55, **characterised in that** the stem (14) is inserted in the three connected plates (748, 750, 752) and it is constrained thereto.

58. Intramedullary nail (10, 110, 210, 310, 410, 510, 610, 710) according to claim 1, **characterised in that** the fractured elongate bone (12) is a femur or a tibia.

59. Application method, in a fractured elongate bone (12), of an intramedullary nail (10, 110, 210, 310, 410, 510, 610, 710) according to claim 1, **characterised in that** it comprises:
a positioning step of said nail (10, 110, 210, 310, 410, 510, 610, 710) in said elongate bone (12), to mend the fracture ;
an operation step of said at least one substantially longitudinal distribution (20) of expansion means for the nail fixation to the bone, said at least one substantially longitudinal distribution (20) of expansion means being operated by said at least one element (22) realised with a shape-memory material.
